(19) **Europäisches Patentamt**
**European Patent Office**
**Office européen des brevets**

(11) **EP 1 744 665 B1**

(12) **EUROPEAN PATENT SPECIFICATION**

(45) Date of publication and mention
of the grant of the patent:
**23.11.2016 Bulletin 2016/47**

(21) Application number: **05732310.7**

(22) Date of filing: **15.04.2005**

(51) Int Cl.:
***A61B 5/00*** (2006.01)

(86) International application number:
**PCT/IB2005/001006**

(87) International publication number:
**WO 2005/099565 (27.10.2005 Gazette 2005/43)**

(54) **PHYSIOLOGICAL EVENT HANDLING SYSTEM, METHOD, AND DEVICE; MONITORING DEVICE AND PROGRAM PRODUCT**

SYSTEM; VERFAHREN UND VORRICHTUNG ZUR BEHANDLUNG EINES PHYSIOLOGISCHEN EREIGNISSES; ÜBERWACHUNGSVORRICHTUNG UND PROGRAMM

SYSTEME, PROCEDE ET DISPOSITIF DE GESTION D'EVENEMENT PHYSIOLOGIQUE ; DISPOSITIF DE SURVEILLANCE ET PRODUIT-PROGRAMME

(84) Designated Contracting States:
**AT BE BG CH CY CZ DE DK EE ES FI FR GB GR HU IE IS IT LI LT LU MC NL PL PT RO SE SI SK TR**

(30) Priority: **15.04.2004 US 825575**

(43) Date of publication of application:
**24.01.2007 Bulletin 2007/04**

(73) Proprietor: **Nokia Technologies Oy**
**02610 Espoo (FI)**

(72) Inventors:
• **HEINONEN, Tomi**
**FIN-33610 Tampere (FI)**

• **KAUPPINEN, Pasi**
**FIN-33610 Tampere (FI)**

(74) Representative: **Swindell & Pearson Limited et al**
**48 Friar Gate**
**Derby DE1 1GY (GB)**

(56) References cited:
**WO-A2-01/47411    WO-A2-01/89362**
**WO-A2-01/89362    DE-A1- 10 219 675**
**US-A- 5 544 661    US-A- 5 720 770**
**US-A- 5 772 586    US-B1- 6 616 606**

**Description**

BACKGROUND OF THE INVENTION

The present invention relates generally to the field of health monitors, and particularly to systems and methods of handling physiological events that may be generated by a physiological monitor.

Handling of said event is disclosed for example in documents WO 01/89362 and WO01/47411.

Health monitoring devices of various types have been used to monitor one or more physiological parameters for a patient. For example, electrocardiograms (ECG) and electroencephalograms (EEG) are capable of measuring electrical functions of the heart and brains, respectively. Also blood pressure, body weight and heart function, for example, can be measured, thereby producing physiological information.

Typically, measured data are not collected and processed centrally, and storage requires manual interaction and/or a reading device specific for these independent systems.

In certain cases, the monitoring device may be mounted on the patient either permanently or on a temporary basis. For example, certain monitors may be implanted into the patient and may monitor certain physiological parameters on a continuous basis. The data from such devices may be recorded. Alternatively, as disclosed in U.S. Patent No. 6,428,475, an instantaneous value of the desired parameters may be displayed on a portable device.

SUMMARY OF THE INVENTION

The present invention is as set out in the independent claims.

One example of the present disclosure relates to a method of handling a physiological event. The method comprises receiving a first signal from a monitor adapted to convey information relating to physiological parameters. The first signal can include Information corresponding to the physiological parameters and an identification of the monitor. The method can also include transmitting a second signal to a network. The second signal can include at least information corresponding to the identification of the monitor.

In another example, there is provided, a system for handling a physiological event including a monitoring device adapted to convey information relating to one or more physiological parameters. The monitoring device is further adapted to transmit a signal including information corresponding at least to an identification of the monitoring device. The system also includes an event handling device adapted to receive signals from the monitoring device, including information corresponding to the identification of the monitoring device. The event handling device is further adapted to transmit a signal including information corresponding to the identification of the monitoring device.

In another example, there is provided a monitoring device. The device includes a monitoring module for conveying information relating to one or more physiological parameters, and a transmitter adapted to transmit a signal. The signal includes information corresponding at least to an identification of the monitoring device

In another example, there is provided an event handling device. The event handling device includes a receiving module adapted to receive signals from a monitor adapted to convey information relating to physiological parameters. The signals include information corresponding to the physiological parameters and an identification of the monitor. The device also includes a transmitting module adapted to transmit including at least information corresponding to the identification of the physiological monitor.

In still another example, there is provided, a program product comprising machine readable program code for causing a machine to perform the following steps: receiving a first signal from a monitor adapted to convey information relating to physiological parameters, and transmitting a second signal to a network. The first signal includes information corresponding to the physiological parameters and an identification of the monitor, and the second signal includes at least information corresponding to the identification of the monitor.

The monitors, monitoring modules, and/or monitor devices can be adapted to detect, sense, or measure the physiological parameters. They can also be adapted to stimulate, intervent, or control physiological functions affecting the physiological

parameters. The physiological parameters can relate to any number of physiological functions such as, for example, heart and/or brain functions.

BRIEF DESCRIPTION OF THE DRAWINGS

FIG. 1 is a diagrammatic illustration of one embodiment of an event handling system according to the invention; and
FIG. 2 is a schematic illustration of one embodiment of certain components of the event handling system of FIG. 1.

DETAILED DESCRIPTION OF THE PREFERRED EMBODIMENTS

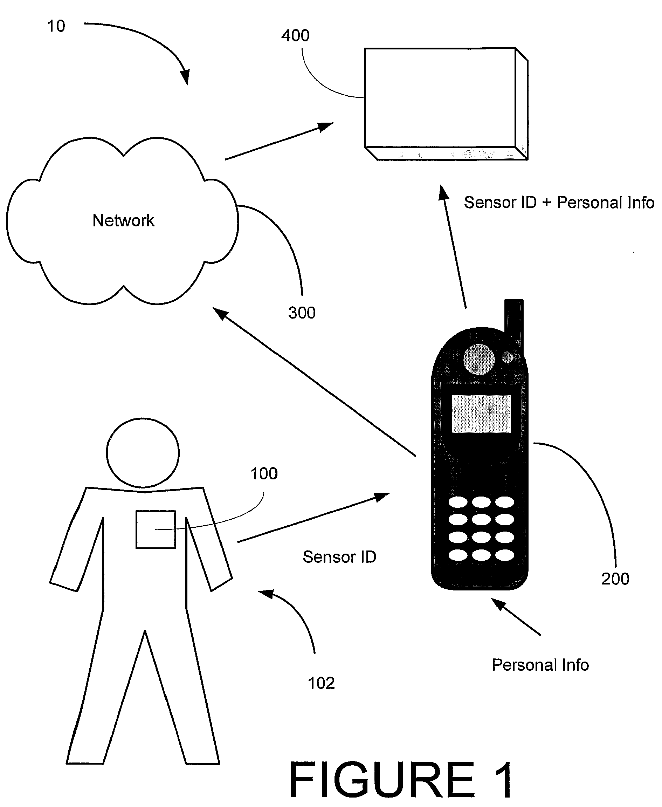

Referring to FIG. 1, an embodiment of an event handling system is diagrammatically illustrated. In the illustrated system 10, a monitoring device 100 is provided which could detect, measure or sense a predetermined set of physiological parameters for a patient 102. The monitoring device can also be adapted to stimulate, intervent, or control physiological functions affecting the physiological parameters. The monitoring device 100 may be externally connected to the patient 102. For example, the monitoring device 100 may be strapped around the chest or the wrist of the user to monitor heart function, for example. Such an arrangement may be used to measure the patient's heart rate, for example, and may be used to detect irregular heartbeat. Other examples of external monitoring devices 100 can include scales, blood pressure measurement devices, etc. In another embodiment, the monitoring device 100 may be implanted into the patient 102.

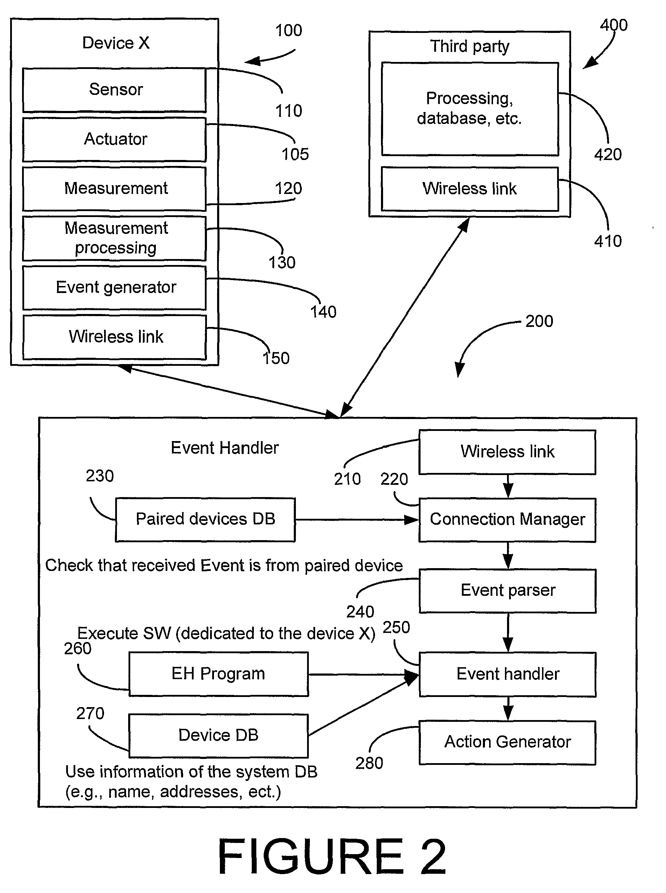

The monitoring device 100 may be provided with an internal power supply, such as a rechargeable battery. Other aspects of the monitoring device 100 are described below with reference to FIG. 2.

The monitoring device 100 is adapted to transmit signals to an event handling device 200. The event handling device 200 is a wireless device, such as a cellular telephone. It will be understood by those skilled in the art that a variety of other devices may also be used as the event handling device 200.

The communication between the devices 100, 200 is accomplished through wireless signals. Wireless protocols, such as Bluetooth or other short range wireless communication technologies, may be used to facilitate the communication. Bluetooth is a standardized communication protocol. Products adapted to use this protocol are qualified for interoperability with all other Bluetooth products. Thus, the use of Bluetooth in the system 10 allows various components to communicate with each other, and eliminates the need for specialized equipment. Thus, a Bluetooth-qualified cellular telephone may be used to receive signals from the monitoring device 100. For additional information on the Bluetooth protocol, reference may be made to the Bluetooth Core Specification, Version 1.2.

The signals transmitted by the monitoring device 100 and received by the event handling device 200 include a variety of information. In particular, the signals contain information which uniquely identifies the monitoring device 100. In turn, this information definitively identifies the identity of the patient to whom the physiological monitoring device 100 belongs. Other information contained in the signals includes various parameters, such as data relating to heart function or brain function.

Information to the patient's heart rate or blood pressure is contained in signals that are transmitted by the monitoring device 100 at predetermined intervals, such as one second.

The signal received by the event handling device 200 is processed to determine the action to be taken by the event handling device 200. The processing of the signal includes verifying the source of the signal. As described below with reference to FIG. 2, the verification includes comparing the identification of the monitoring device 100 with identifications of various devices stored in a database. The database is contained in the event handling device 200.

The processing includes determining whether the information contained in the signal requires notification to a third party. For example, the signal may include information indicating that the patient requires immediate medical attention or the monitoring device 100 requires attention due to a malfunction or a low battery, for example. This information may take the form of a flag contained in the signal or raw physiological data which may be compared against thresholds stored in the event handling device 200.

When the event handling device 200 determines that a third party requires notification, a signal is transmitted from the event handling device 200 to a communication network 300, through which a third party, such as a medical facility 400, is notified. The network 300 may be a wireless communication network, such as a cellular network. The signal transmitted by the event handling device 200 may include the information in the signal received by the event handling device 200 from the monitoring device 100. Additional information may be added by the event handling device 200, such as an identification of the event handling device 200. Further, the event handling device 200 may re-format the information to more readily convey the information to an operator at the medical facility 400, for example.

Thus, the third party receiving the signal from the event handling device 200, such as the medical facility 400, can quickly identify the event handling device 200 and evaluate the need for urgent medical attention. Since the signal also contains information corresponding to the identification of the monitoring device 100, the third party can, with certainty, identify the patient associated with the signal received from the event handling device 200.

The monitoring device 100 is configured, in certain situations, to broadcast a general emergency signal. In addition, software enabling minimal event handling capabilities can be included in various mobile devices such that these mobile devices are capable of receiving and processing the general emergency signal. In this situation, mobile devices within communication range of the monitoring device 100 can be configured to relay this message, through a communications network, to an emergency response facility. For example, if the patient 102 were to experience a cardiac arrest, the monitoring device 100 could detect this emergency situation and broadcast an emergency signal and all mobile devices equipped with the minimal event handling capabilities could detect and process the message.

More particularly, mobile devices, such as mobile telephones, may be configured to include at least event handling capabilities sufficient to recognize and act upon the emergency signal. The broadcast emergency signal could be of a very specific configuration such that the emergency response facility could recognize the location of the patient (or monitoring device 100) such as through the use of global positioning information. As such, the emergency response facility could dispatch a response team to the patient's location.

In other embodiments of the disclosure a two-way communication channel can be opened up with the monitoring device 100 and used to maintain the monitoring device 100. As explained in more detail below, the monitoring device 100 can be configured to relay it's serial number or other identifying information. The system 10 can be configured to download software, such as device drivers or database information, to specific monitoring devices 100 based on predetermined maintenance schedules or on the specific functionality of the monitoring device 100. In addition, the two-way communication channel can be used to allow communication between monitoring devices 100.

FIG. 2 provides a schematic illustration of certain components of the system 10 in greater detail. As described above with reference to FIG. 1, the monitoring device 100 is adapted to transmit signals to the event handling device 200. A double arrow is shown in FIG. 2 to indicate that, in certain embodiments, instructions may be transmitted to the monitoring device 100 through the communication link with the event handling device 200. Similarly, a double arrow between the event handling device 200 and the third party 400 indicates a two-way communication link.

An embodiment of the monitoring device 100 will now be described with reference to FIG. 2. The monitoring device 100 may include one or more sensors 110 or measurement devices 120 to detect, sense or measure various physiological parameters. The monitoring device 100 may also include one or more actuators 105 for stimulating, interventing, and/or controlling physiological functions affecting the physiological parameters. Such actuators 105, sensors 110, and measurement devices 120 are well known to those skilled in the art.

A measurement processing module 130 is provided in the physiological monitoring device 100 to process the data from the sensors 110 or measurement devices 120. The measurement processing module 130 may determine whether an event has occurred requiring transmission of a signal. An event may be predefined as, for example, the expiration of an interval or one or more physiological parameters satisfying a predetermined criteria, such as maximum blood pressure. Events can originate based on a variety of detected stimuli. For example, events can originate from organs, such as the heart, brain, muscles, etc.) producing measurable electrical currents, potentials, or magnetic fields. The electrical or chemical properties of body fluids as affected by such factors as blood sugar, Cerebro spinal fluid conductivity, urine, etc, or mechanical movement or orientation of the body or body structures, such as organs, muscles, or body orientation, can also generate events. Even acoustic signals or thermal information about the body or body structures or fluids can generate events.

In one example, an event may be indicated when the measurement processing module 130 determines that the data indicates a fibrillation detected by the monitoring device 100, which may be a heart pacemaker. Thus, the measurement processing module 130 may conclude either that no event has occurred, in which case it continues to receive data from the sensors 110 or measurement devices 120, or that an event has occurred, in which case it delivers certain information to an event generator 140.

The event generator 140 assembles a message to be transmitted as a signal by the monitoring device 100. In one example, the message may include the following information: sender identification, sender type, time/date, physiological parameter, value of physiological parameter, and an end mark. The sender identification may be, for example, a serial number of the monitoring device 100 or an identifier of the patient to whom the monitoring device 100 belongs. In the case where the monitoring device 100 includes multiple sensors, a sensor ID may also be included. In the above-described fibrillation example, the message may appear as:

$$SERIAL\ NO + PACEMAKER\ MODEL\ ABC + SENSOR\ ID + DATE + TIME$$

$$+FATAL\ CARDIAC\ SYMPTOM + ACUTE\ FIBRILLATION + END$$

The message is then transmitted by the monitoring device 100 through a communication link, such as a wireless link 150.

The message from the monitoring device 100 is received by the event handling device 200. As noted above, in a particular embodiment, the communication between the monitoring device 100 and the event handling device 200 can be through a wireless link using the Bluetooth protocol. The signal containing the message is received by the event handling device 200 through a wireless link 210. A connection manager module 220 is provided to initially process the signal. The initial processing 220 includes determining whether the signal is received from a valid source. In this regard, the event handling device 200 may include a list or database 230 of paired devices with which the event handling device 200 can validly communicate. This can ensure that signals from other devices or similar devices belonging to nearby persons are not mistakenly processed.

Once the signal is determined to be from a valid paired device, the message in the signal is parsed by an event parser 240. The event parser 240 may be adapted to isolate certain information for processing by additional modules, while retaining other information for inclusion in a message to be transmitted by the event handling device 200. In this regard, the event parser 240 may isolate information relating to the physiological parameters for processing by an event handler module 250. The event handler module 250 executes a software algorithm 260 stored in the event handling device 200 to determine the appropriate action. The software algorithm 260 may be adapted to compare the physiological parameters to information stored in a device database 270, such as threshold levels for various actions.

In the above-described fibrillation example, the software algorithm may appear as follows:

```
If "Measurement Parameter"="BATTERY LOW"
      Then Action= "Display Corresponding Message in EH Display"
If "Measurement Parameter"= "FATAL"
      Then Action= "Send Message to Hospital Server, Measurement
            Parameter, Measurement Value"
If "Measurement Value"= "ACUTE FIBRILLATION"
      Then Auction= "Ask device X to activate defibrillation "
```

Thus, the event handler 250 can determine an appropriate action to be taken. The appropriate action may include transmitting a message indicating a medical emergency, malfunction of the monitoring device 100 or mere notification of the data relating to physiological parameters. In some cases, the appropriate action may be to take no action at all.

The event handler 250 can then transmit instruction to an action generator 280 corresponding to the action determined to be appropriate. In response, the action generator 280 generates a message to be transmitted to the third party 400. In this regard, the message generated by the action generator 280 may include all or part of the information received in the signal from the monitoring device 100 as well as additional information, such as identity of the event handling device 200. The generated message may also be formatted to be readily understandable by the third party 400. The formatted message is then sent by the action generator 280 to the wireless link 210 for transmission to the third party 400.

As noted above, the communication between the event handling device 200 and the third party 400 may be through a communication network, which may be a wireless network such as a cellular network. The signal from the event handling device 200 is received by a wireless link 410 in the facilities of the third party 400. The signal may then be processed by one or more modules 420 to be displayed, for example, to an operator, nurse or physician. Since the message contains information corresponding to the identification of the monitoring device 100, the patient's identity can be determined with great certainty.

The above-described system and associated components may have a variety of applications. Some exemplary applications are provided below.

Cardiac Implant

The event handling device or a hospital may control the implanted monitoring device. If there was a malfunction in the implant, a notice may be given to the patient, who then has a possibility to respond to the situation. Routine visits to a physician for pacemaker control and maintenance may be reduced. Also, information may be available to the patient outside the hospital environment (e.g., when exercising there could be a notice to slow down in order to lower the heartrate.). If pacemaker detects changes in the heart, information would be available immediately, rather than waiting until the next routine check-up, which may be too late. A cardiac implant may also use defibrillation or another form of stimulation to affect or treat potential or actual heart problems.

Epilepsy Implant

An epilepsy implant could recognize an impending or existing epileptic seizure. Epileptic seizures can occur without warning, thus severely restricting various aspects of a patient's life. Detection of an impending seizure may be delivered to the patient or a third party. An epilepsy implant may also be used to actively suppress an impending seizure.

Drug Dosage Implant

Almost real-time information about the dosage used, changes in it and the status of the device may be made available to the patient or the third party.

Blood Pressure Monitor

Measured values may be automatically stored in the memory of the event handling device, from which they can be accessed and displayed in an informative way (e.g. trend curves). Reliable and simple follow-up, the frequency of follow-up in hospital is thus dictated by actual needs. Physician may be immediately notified if necessary.

Dialysis Monitor

Patients requiring dialysis operation periodically enter dialysis on a scheduled basis which, for conservative reasons, tends to be more often than needed. An implant can track a need for dialysis, and the data can be monitored by a medical facility. The medical facility can call the patient for treatment only when needed.

**Claims**

1.  A method comprising:

    receiving, at a mobile wireless event handling device (200) at a predetermined interval, a first signal via a wireless communication link in a first network from a monitoring device (100) configured to convey information relating to physiological parameters, the first signal including information corresponding to the physiological parameters and an identification of the monitoring device (100);
    processing the first signal at the mobile wireless event handling device, the processing comprising determining whether the first signal is received from a valid source;
    receiving, at the mobile wireless event handling device (200), a broadcast general emergency signal from the monitoring device (100); and
    in response to said receiving the broadcast general emergency signal, determining to transmit, at the mobile wireless event handling device (200), to a target (400), a second signal via a second network, the second signal including at least information corresponding to the identification of the monitoring device (100);
    wherein determining whether the first signal is received from a valid source comprises determining, from a database of paired devices within the mobile wireless event handling device (200), whether communication of the mobile wireless event handling device (200) with the monitoring device (100) is valid.

2.  The method of claim 1, wherein the monitoring device (100) is an implant.

3.  The method of claim 1 or claim 2, wherein the monitoring device (100) is configured to detect, sense, or measure the physiological parameters.

4.  The method of any preceding claim, wherein the monitoring device (100) is configured to stimulate, intervent, or control physiological functions affecting the physiological parameters.

5.  The method of any preceding claim, wherein the physiological parameters relate to heart function and/or brain function.

6.  The method of claim 1 , wherein the first network and the second network are different networks.

7.  The method of claim 1 or claim 6, wherein the second network is a cellular network.

8.  The method of any preceding claim, further comprising:

    processing the first signal prior to transmitting the second signal.

9.  The method of claim 8 , wherein processing further comprises:

    verifying a source of the first signal;
    identifying an event associated with the first signal and related to the physiological parameters; and
    determining a target (400) for the second signal.

10. The method of any preceding claim, wherein when the mobile wireless event handling device (200) determines that a third party requires notification, transmitting from the mobile wireless event handling device (200) to the third party.

11. The method of any preceding claim, wherein the second signal further includes identification of the mobile wireless event handling device (200).

**12.** The method of claim 1 , wherein the general broadcast emergency signal is received by all mobile wireless event handling devices (200) within communication range of the monitoring device (100).

**13.** A mobile wireless event handling device (200), comprising:

a receiving module configured to receive signals via a wireless communication link over a first network, at a predetermined interval, from a monitoring device (100) configured to convey information relating to physiological parameters, the signals including information corresponding to the physiological parameters and an identification of the monitoring device (100);
a connection manager module configured to process the signals received from the monitoring device (100), in which the process comprises determining whether the signals are received from a valid source;

**characterised by** further comprising:

receiving means configured to receive a broadcast general emergency signal from the monitoring device; and
a transmitting module configured to transmit signals including at least information corresponding to the identification of the monitoring device (100), to a target (400) via a second network in response to said receiving means receiving the broadcast general emergency signal;
wherein determining whether the signals are received from a valid source comprises determining, from a database (230) of paired devices within the mobile wireless event handling device (200), whether communication of the mobile wireless event handling device (200) with the monitoring device (100) is valid.

**14.** The device of claim 13 , wherein the monitoring device (100) is configured to detect, sense, or measure the physiological parameters.

**15.** The device of claim 13 or 14 , wherein the monitoring device (100) is configured to stimulate, intervent, or control physiological functions affecting the physiological parameters.

**16.** The device of any of claims 13 to 15 wherein the physiological parameters relate to heart function and/or brain function.

**17.** The device of any of claims 13 to 16 , wherein the transmitting module is configured to transmit signals when one or more physiological parameters satisfies a predetermined criteria.

**18.** The device of any of claims 13 to 17 wherein the first network and the second network are different networks.

**19.** The device of any of claims 13 to 18, further comprising:

a data processing module configured to verify a source of signals received by the receiving module, the data processing module being further configured to identify an event associated with the signals received by the receiving module and to determine a target for signals transmitted by the transmitting module.

**20.** A system for handling an event, comprising the mobile wireless event handling device (200) of any of claims 13 to 19 , and further comprising a monitoring device (100) configured to convey information relating to one or more physiological parameters, the monitoring device (100) being further configured to transmit a signal via the first network, the signal including information corresponding at least to an identification of the monitoring device (100), and to broadcast a general emergency signal adapted for receipt by the mobile wireless device (200).

**21.** The system of claim 20 wherein the monitoring device (100) is implantable in a human body.

**22.** The system of any of claims 20 to 21 , wherein the monitoring device (100) is configured to transmit a signal when one or more physiological parameters satisfies a predetermined criteria.

**23.** The system of any of claims 20 to 22 , wherein the monitoring device (100) is configured to transmit signals on a substantially continuous basis.

**24.** A computer program comprising program code means configured to perform the method of any of claims 1 to 17 when the program is run on a data processing device being a part of the mobile wireless event handling device (200) according to any of the claims 13-23.

**Patentansprüche**

1. Verfahren, das Folgendes umfasst:

   Empfangen, in einer mobilen drahtlosen Ereignishandhabungsvorrichtung (200) in einem zuvor festgelegten Intervall, eines ersten Signals über eine drahtlose Kommunikationsverbindung in einem ersten Netz von einer Überwachungsvorrichtung (100), die dafür konfiguriert ist, Informationen bezüglich physiologischer Parameter zu transportieren, wobei das erste Signal Informationen enthält, die den physiologischen Parametern und einer Identifikation der Überwachungsvorrichtung (100) entsprechen;
   Verarbeiten des ersten Signals in der mobilen drahtlosen Ereignishandhabungsvorrichtung, wobei das Verarbeiten das Bestimmen umfasst, ob das erste Signal von einer gültigen Quelle empfangen wird;
   Empfangen, in der mobilen drahtlosen Ereignishandhabungsvorrichtung (200), eines rundgesendeten allgemeinen Notfallsignals von der Überwachungsvorrichtung (100); und
   in Reaktion auf das Empfangen des rundgesendeten allgemeinen Notfallsignals, Bestimmen, in der mobilen drahtlosen Ereignishandhabungsvorrichtung (200), ein zweites Signal über ein zweites Netz an ein Ziel (400) zu senden, wobei das zweite Signal mindestens Informationen enthält, die der Identifikation der Überwachungsvorrichtung (100) entsprechen;
   wobei das Bestimmen, ob das erste Signal von einer gültigen Quelle empfangen wird, umfasst, anhand einer Datenbank gepaarter Geräte innerhalb der mobilen drahtlosen Ereignishandhabungsvorrichtung (200) zu bestimmen, ob eine Kommunikation der mobilen drahtlosen Ereignishandhabungsvorrichtung (200) mit der Überwachungsvorrichtung (100) gültig ist.

2. Verfahren nach Anspruch 1, wobei die Überwachungsvorrichtung (100) ein Implantat ist.

3. Verfahren nach Anspruch 1 oder Anspruch 2, wobei die Überwachungsvorrichtung (100) dafür konfiguriert ist, die physiologischen Parameter zu detektieren, abzufühlen oder zu messen.

4. Verfahren nach einem der vorangehenden Ansprüche, wobei die Überwachungsvorrichtung (100) dafür konfiguriert ist, physiologische Funktionen, welche die physiologischen Parameter beeinflussen, zu stimulieren, zu beeinflussen oder zu steuern.

5. Verfahren nach einem der vorangehenden Ansprüche, wobei die physiologischen Parameter Herzfunktion und/oder Gehirnfunktion betreffen.

6. Verfahren nach Anspruch 1, wobei das erste Netz und das zweite Netz verschiedene Netze sind.

7. Verfahren nach Anspruch 1 oder Anspruch 6, wobei das zweite Netz ein Mobilfunknetz ist.

8. Verfahren nach einem der vorangehenden Ansprüche, das des Weiteren Folgendes umfasst:

   Verarbeiten des ersten Signals vor dem Senden des zweiten Signals.

9. Verfahren nach Anspruch 8, wobei das Verarbeiten des Weiteren Folgendes umfasst:

   Verifizieren einer Quelle des ersten Signals;
   Identifizieren eines Ereignisses, das dem ersten Signal zugeordnet ist und mit den physiologischen Parametern im Zusammenhang steht; und
   Bestimmen eines Ziels (400) für das zweite Signal.

10. Verfahren nach einem der vorangehenden Ansprüche, wobei, wenn die mobile drahtlose Ereignishandhabungsvorrichtung (200) bestimmt, dass ein Dritter eine Benachrichtigung verlangt, Senden von der mobilen drahtlosen Ereignishandhabungsvorrichtung (200) an den Dritten.

11. Verfahren nach einem der vorangehenden Ansprüche, wobei das zweite Signal des Weiteren eine Identifikation der mobilen drahtlosen Ereignishandhabungsvorrichtung (200) enthält.

12. Verfahren nach Anspruch 1, wobei das allgemeine rundgesendete Notfallsignal durch alle mobilen drahtlosen Ereignishandhabungsvorrichtungen (200) innerhalb der Kommunikationsreichweite der Überwachungsvorrichtung (100) empfangen wird.

13. Mobile drahtlose Ereignishandhabungsvorrichtung (200), die Folgendes umfasst:

ein Empfangsmodul, das dafür konfiguriert ist, Signale über eine drahtlose Kommunikationsverbindung über ein erstes Netz in einem zuvor festgelegten Intervall von einer Überwachungsvorrichtung (100) zu empfangen, die dafür konfiguriert ist, Informationen bezüglich physiologischer Parameter zu transportieren, wobei die Signale Informationen enthalten, die den physiologischen Parametern und einer Identifikation der Überwachungsvorrichtung (100) entsprechen;
ein Verbindungsmanagermodul, das dafür konfiguriert ist, die von der Überwachungsvorrichtung (100) empfangenen Signale zu verarbeiten, wobei der Prozess umfasst zu bestimmen, ob die Signale von einer gültigen Quelle empfangen werden;

**dadurch gekennzeichnet, dass** sie des Weiteren Folgendes umfasst:

ein Empfangsmittel, das dafür konfiguriert ist, ein rundgesendetes allgemeines Notfallsignal von der Überwachungsvorrichtung zu empfangen; und
ein Sendemodul, das dafür konfiguriert ist, Signale, die mindestens Informationen enthalten, die der Identifikation der Überwachungsvorrichtung (100) entsprechen, in Reaktion darauf, dass das Empfangsmittel das rundgesendete allgemeine Notfallsignal empfängt, über ein zweites Netz an ein Ziel (400) zu senden;
wobei das Bestimmen, ob die Signale von einer gültigen Quelle empfangen werden, umfasst, anhand einer Datenbank (230) gepaarter Geräte innerhalb der mobilen drahtlosen Ereignishandhabungsvorrichtung (200) zu bestimmen, ob eine Kommunikation der mobilen drahtlosen Ereignishandhabungsvorrichtung (200) mit der Überwachungsvorrichtung (100) gültig ist.

14. Vorrichtung nach Anspruch 13, wobei die Überwachungsvorrichtung (100) dafür konfiguriert ist, die physiologischen Parameter zu detektieren, abzufühlen oder zu messen.

15. Vorrichtung nach Anspruch 13 oder 14, wobei die Überwachungsvorrichtung (100) dafür konfiguriert ist, physiologische Funktionen, welche die physiologischen Parameter beeinflussen, zu stimulieren, zu beeinflussen oder zu steuern.

16. Vorrichtung nach einem der Ansprüche 13 bis 15, wobei die physiologischen Parameter Herzfunktion und/oder Gehirnfunktion betreffen.

17. Vorrichtung nach einem der Ansprüche 13 bis 16, wobei das Sendemodul dafür konfiguriert ist, Signale zu senden, wenn ein oder mehrere physiologische Parameter ein zuvor festgelegtes Kriterium erfüllen.

18. Vorrichtung nach einem der Ansprüche 13 bis 17, wobei das erste Netz und das zweite Netz verschiedene Netze sind.

19. Vorrichtung nach einem der Ansprüche 13 bis 18, die des Weiteren Folgendes umfasst:

ein Datenverarbeitungsmodul, das dafür konfiguriert ist, eine Quelle von Signalen zu verifizieren, die durch das Empfangsmodul empfangen werden, wobei das Datenverarbeitungsmodul des Weiteren dafür konfiguriert ist, ein Ereignis zu identifizieren, das mit den Signalen im Zusammenhang steht, die durch das Empfangsmodul empfangen werden, und ein Ziel für durch das Sendemodul gesendete Signale zu bestimmen.

20. System zum Handhaben eines Ereignisses, das die mobile drahtlose Ereignishandhabungsvorrichtung (200) nach einem der Ansprüche 13 bis 19 umfasst, und des Weiteren eine Überwachungsvorrichtung (100) umfasst, die dafür konfiguriert ist, Informationen bezüglich eines oder mehrerer physiologischer Parameter zu transportieren, wobei die Überwachungsvorrichtung (100) des Weiteren dafür konfiguriert ist, ein Signal über das erste Netz zu senden, wobei das Signal Informationen enthält, die mindestens einer Identifikation der Überwachungsvorrichtung (100) entsprechen, und ein allgemeines Notfallsignal rundzusenden, das dafür ausgelegt ist, durch die mobile drahtlose Vorrichtung (200) empfangen zu werden.

21. System nach Anspruch 20, wobei die Überwachungsvorrichtung (100) in den Körper eines Menschen implantiert werden kann.

**22.** System nach einem der Ansprüche 20 und 21, wobei die Überwachungsvorrichtung (100) dafür konfiguriert ist, ein Signal zu senden, wenn ein oder mehrere physiologische Parameter ein zuvor festgelegtes Kriterium erfüllen.

**23.** System nach einem der Ansprüche 20 bis 22, wobei die Überwachungsvorrichtung (100) dafür konfiguriert ist, Signale auf im Wesentlichen kontinuierlicher Basis zu senden.

**24.** Computerprogramm, das ein Programmcode-Mittel umfasst, das dafür konfiguriert ist, das Verfahren nach einem der Ansprüche 1 bis 17 auszuführen, wenn das Programm auf einer Datenverarbeitungsvorrichtung abläuft, die Teil der mobilen drahtlosen Ereignishandhabungsvorrichtung (200) nach einem der Ansprüche 13-23 ist.

## Revendications

**1.** Procédé comprenant :

la réception, au niveau d'un dispositif de gestion d'événement sans fil mobile (200) et à un intervalle prédéterminé, d'un premier signal via une liaison de communication sans fil dans un premier réseau, depuis un dispositif de surveillance (100) configuré pour convoyer des informations concernant des paramètres physiologiques, le premier signal contenant des informations correspondant aux paramètres physiologiques et une identification du dispositif de surveillance (100) ;
le traitement du premier signal au niveau du dispositif de gestion d'événement sans fil mobile, le traitement comprenant le fait de déterminer si le premier signal est reçu depuis une source valide ;
la réception, au niveau du dispositif de gestion d'événement sans fil (200), d'un signal d'urgence générale de diffusion provenant du dispositif de surveillance (100) ; et
en réponse à ladite réception du signal d'urgence générale de diffusion, la détermination d'une transmission, au niveau du dispositif de gestion d'événement sans fil mobile (200), vers une cible (400), d'un deuxième signal via un deuxième réseau, le deuxième signal comprenant au moins des informations correspondant à l'identification du dispositif de surveillance (100) ;
dans lequel le fait de déterminer si le premier signal est reçu depuis une source valide comprend le fait de déterminer, à partir d'une base de données de dispositifs appariés à l'intérieur du dispositif de gestion d'événement sans fil mobile (200), si la communication du dispositif de gestion d'événement sans fil mobile (200) avec le dispositif de surveillance (100) est valide.

**2.** Procédé selon la revendication 1, dans lequel le dispositif de surveillance (100) est un implant.

**3.** Procédé selon la revendication 1 ou la revendication 2, dans lequel le dispositif de surveillance (100) est configuré pour détecter, capter ou mesurer des paramètres physiologiques.

**4.** Procédé selon l'une quelconque des revendications précédentes, dans lequel le dispositif de surveillance (100) est configuré pour stimuler, intervenir sur, ou contrôler des fonctions physiologiques affectant les paramètres physiologiques.

**5.** Procédé selon l'une quelconque des revendications précédentes, dans lequel les paramètres physiologiques concernent la fonction cardiaque et/ou la fonction cérébrale.

**6.** Procédé selon la revendication 1, dans lequel le premier réseau et le deuxième réseau sont des réseaux différents.

**7.** Procédé selon la revendication 1 ou la revendication 6, dans lequel le deuxième réseau est un réseau cellulaire.

**8.** Procédé selon l'une quelconque des revendications précédentes, comprenant en outre le traitement du premier signal avant la transmission du deuxième signal.

**9.** Procédé selon la revendication 8, dans lequel le traitement comprend en outre :

la vérification d'une source du premier signal ;
l'identification d'un événement associé au premier signal et concernant les paramètres physiologiques ; et
la détermination d'une cible (400) pour le deuxième signal.

**10.** Procédé selon l'une quelconque des revendications précédentes, dans lequel, quand le dispositif de gestion d'événement sans fil mobile (200) détermine qu'une tierce partie requiert une notification, transmettre depuis le dispositif de gestion d'événement sans fil mobile (200) à la tierce partie.

**11.** Procédé selon l'une quelconque des revendications précédentes, dans lequel le deuxième signal comprend en outre l'identification du dispositif de gestion d'événement sans fil mobile (200).

**12.** Procédé selon la revendication 1, dans lequel le signal d'urgence de diffusion générale est reçu par tous les dispositifs de gestion d'événement sans fil (200) dans la plage de communication du dispositif de surveillance (100).

**13.** Dispositif de gestion d'événement sans fil mobile (200) comprenant :

un module de réception configuré pour recevoir des signaux via une liaison de communication sans fil sur un premier réseau, à un intervalle prédéterminé, depuis un dispositif de surveillance (100) configuré pour convoyer des informations concernant des paramètres physiologiques, les signaux comprenant des informations concernant les paramètres physiologiques et une identification du dispositif de surveillance (100) ;
un module gestionnaire de connexion configuré pour traiter les signaux reçus depuis le dispositif de surveillance (100), le procédé comprenant le fait de déterminer si les signaux sont reçus depuis une source valide ;

**caractérisé en ce qu'**il comprend en outre :

des moyens de réception configurés pour recevoir un signal d'urgence générale de diffusion depuis le dispositif de surveillance ; et
un module de transmission configuré pour transmettre à une cible (400) des signaux comprenant au moins des informations correspondant à l'identification du dispositif de surveillance (100), via un deuxième réseau en réponse auxdits moyens de réception recevant le signal d'urgence générale de diffusion ;
dans lequel le fait de déterminer si les signaux sont reçus depuis une source valide comprend le fait de déterminer, à partir d'une base de données (230) de dispositifs appariés à l'intérieur du dispositif de gestion d'événement sans fil mobile (200), si la communication du dispositif de gestion d'événement sans fil mobile (200) avec le dispositif de surveillance (100) est valide.

**14.** Dispositif selon la revendication 13, dans lequel le dispositif de surveillance (100) est configuré pour détecter, capter ou mesurer les paramètres physiologiques.

**15.** Dispositif selon la revendication 13 ou 14, dans lequel le dispositif de surveillance (100) est configuré pour stimuler, intervenir sur, ou contrôler des fonctions physiologiques affectant les paramètres physiologiques.

**16.** Dispositif selon l'une quelconque des revendications 13 à 15, dans lequel les paramètres physiologiques concernent la fonction cardiaque et/ou la fonction cérébrale.

**17.** Dispositif selon l'une quelconque des revendications 13 à 16, dans lequel le module de transmission est configuré pour transmettre des signaux quand un ou plusieurs paramètres physiologiques satisfont à des critères prédéterminés.

**18.** Dispositif selon l'une quelconque des revendications 13 à 17, dans lequel le premier réseau et le deuxième réseau sont des réseaux différents.

**19.** Dispositif selon l'une quelconque des revendications 13 à 18, comprenant en outre un module de traitement de données configuré pour vérifier une source de signaux reçus par le module de réception, le module de traitement de données étant en outre configuré pour identifier un événement associé aux signaux reçus par le module de réception et pour déterminer une cible pour les signaux transmis par le module de transmission.

**20.** Système pour gérer un événement, comprenant le dispositif de gestion d'événement sans fil mobile (200) de l'une quelconque des revendications 13 à 19, et comprenant en outre un dispositif de surveillance (100) configuré pour convoyer des informations concernant un ou plusieurs paramètres physiologiques, le dispositif de surveillance (100) étant en outre configuré pour transmettre un signal via le premier réseau, le signal contenant des informations correspondant au moins à une identification du dispositif de surveillance (100), et pour diffuser un signal d'urgence générale adapté pour être reçu par le dispositif sans fil mobile (200).

**21.** Système selon la revendication 20, dans lequel le dispositif de surveillance (100) est implantable dans un corps humain.

**22.** Système selon l'une quelconque des revendications 20 et 21, dans lequel le dispositif de surveillance (100) est configuré pour transmettre un signal quand un ou plusieurs paramètres physiologiques satisfont à des critères prédéterminés.

**23.** Système selon l'une quelconque des revendications 20 à 22, dans lequel le dispositif de surveillance (100) est configuré pour transmettre des signaux sur une base pratiquement continue.

**24.** Programme informatique comprenant des moyens formant code informatique configurés pour effectuer le procédé de l'une quelconque des revendications 1 à 17 quand le programme est exécuté sur un dispositif de traitement de données qui fait partie du dispositif de gestion d'événement sans fil mobile (200) selon l'une quelconque des revendications 13 à 23.

10

400

Network

Sensor ID + Personal Info

300

100

Sensor ID

200

102

Personal Info

# FIGURE 1

**Device X** 100

| Sensor | 110 |
| Actuator | 105 |
| Measurement | 120 |
| Measurement processing | 130 |
| Event generator | 140 |
| Wireless link | 150 |

**Third party** 400

| Processing, database, etc. | 420 |
| Wireless link | 410 |

200

**Event Handler** 210

230

Paired devices DB

Check that received Event is from paired device

Execute SW (dedicated to the device X) 250

260

EH Program

270

Device DB

Use information of the system DB
(e.g., name, addresses, ect.)

Wireless link 220

Connection Manager

Event parser 240

Event handler

Action Generator 280

# FIGURE 2

**EP 1 744 665 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 0189362 A
- WO 0147411 A
- US 6428475 B